# EUROPEAN PATENT SPECIFICATION

(11) **EP 4 284 312 B1**
(45) Date of publication and mention of the grant of the patent: **29.01.2025**
(21) Application number: 22704514.3
(22) Date of filing: 31.01.2022
(51) Int. Cl.: A61F 5/56, A61B 5/03, A61B 5/00, A61B 5/22

(54) **DEVICE FOR DETERMINING TONGUE POSITION BY MEASURING NEGATIVE PRESSURE IN THE ORAL CAVITY, FOR MEASURING INHALATION PRESSURE IN THE NASOPHARYNGEAL CAVITY, AND ASSOCIATED TERMINAL**
VORRICHTUNG ZUR BESTIMMUNG DER ZUNGENPOSITION DURCH MESSUNG DES UNTERDRUCKS IN DER MUNDHÖHLE ZUR MESSUNG DES INHALATIONSDRUCKS IN DER NASOPHARYNGEALHÖHLE UND ZUGEHÖRIGES ENDGERÄT
DISPOSITIF POUR DÉTERMINER LA POSITION DE LA LANGUE PAR MESURE DE LA PRESSION NÉGATIVE DANS LA CAVITÉ BUCCALE, POUR MESURER LA PRESSION D'INHALATION DANS LA CAVITÉ NASOPHARYNGÉE, ET TERMINAL ASSOCIÉ

(30) Priority: 29.01.2021 US 202163143399 P; 16.03.2021 ES 202130232
(43) Date of publication of application: 06.12.2023
(73) Proprietor: Kahn, Sandra Vivian, San Mateo, CA 94402 (US); Engelke, Wilfried Gerhard Hermann, 37130 Gleichen (DE)
(72) Inventor: Kahn, Sandra Vivian, San Mateo, CA 94402 (US); Engelke, Wilfried Gerhard Hermann, 37130 Gleichen (DE)
(74) Representative: Isern Patentes y Marcas S.L.
(86) International application number: PCT/EP2022/052245
(87) International publication number: WO 2022/162229

(56) References cited:
- EP-A1- 2 135 552
- EP-A1- 2 351 543
- EP-A1- 2 740 404
- WO-A1-2018/126295
- WO-A1-2019/195579
- JP-A- 2001 275 994
- US-A1- 2009 186 324
- US-A1- 2011 220 124
- US-A1- 2012 017 917
- US-A1- 2014 190 489
- OFUSA WATARU ET AL: "Use of barometric pressure and electromyography measurement techniques to elucidate the mechanisms by which bolus passes from the oral cavity to the oropharynx during swallowing", PHYSIOLOGY AND BEHAVIOR, ELSEVIER SCIENCE LTD., OXFORD, GB, vol. 226, 4 August 2020 (2020-08-04), XP086257340, ISSN: 0031-9384, [retrieved on 20200804], DOI: 10.1016/J.PHYSBEH.2020.113115

## Description

### TECHNICAL FIELD

The present invention falls within intraoral devices, specifically in the sector of vacuum-activation intraoral devices, and more specifically intraoral devices that facilitate the creation of vacuum pressure in the oral cavity and its measurement based on the tongue movement produced by swallowing.

Likewise, the device of the invention relates to measuring inhalation pressure in the nasopharyngeal cavity.

### BACKGROUND OF THE INVENTION

The need to keep the tongue in the correct position to prevent, for example, loud breathing such as snoring, which occurs when breathing through the mouth during sleep when the tongue partially blocks the airways, is known in the clinical world. For this reason, it is necessary for the tongue to remain in a forward position which ensures that these passages are kept clear or unobstructed.

In addition, apart from the discomfort caused by snoring during the stages of sleep, obstruction of the airways can represent a source of health problems, causing a cut in the air supply to the lungs, commonly known as apnoea. This decreases the amount of oxygen carried by the blood to the brain, causing disruptive sleep patterns and resulting in chronic fatigue that can cumulatively cause damage.

That is why it is necessary for many people to reduce or eliminate these incorrect positions of the tongue inside the oral cavity, avoiding the possible respiratory problems that it may cause.

There are numerous documents in the state of the art that refer to devices intended to correctly position the tongue.

One example of said documents is patent US5373859, which describes a device for retaining the tongue and reducing snoring, comprising a housing that defines a flexible vacuum reservoir and a rigid flange that completely surrounds the housing and makes contact with the user's face around the entire perimeter of the mouth. The opening of the reservoir fits over the tongue and holds the tongue under negative pressure created with the device.

Document US4169473 also belongs to the state of the art, which discloses a device to be placed inside a user's mouth, with the aim of preventing snoring and/or bruxism, which is configured as a moulded body that provides an externally located portion, and a portion placed inside the user's oral cavity, with dental engaging arches and a rearwardly-opening central socket for cooperating with the forward portion of the tongue, forcing it to be placed in the front portion of the mouth, increasing the unobstructed dimension of the breathing passage.

Document WO2018076088 is also found in the state of the art and proposes an intraoral device for creating a sensation of contact between the teeth, which must be applied to the user's upper and/or lower palate, and which comprises a contact means designed to be associated with at least one portion of one to four posterior teeth, with the aim of creating a sensation of contact for the user by means of the contact between the very contact means and at least one portion of the opposing dental area.

Document ES1067430 also belongs to the state of the art and describes an intraoral device for preventing snoring and/or apnoea, which is made up of a main body, which incorporates a flexible fin on the rear portion thereof, which retains the movement of the tongue, preventing it from moving backwards and therefore preventing the user from snoring.

Further documents also belonging to the sate of the art are US 2012/017917 and US 2011/220124.

As can be seen, there are numerous intraoral devices in the state of the art intended to position the tongue inside the oral cavity in such a way that ensures that the user's airways are not obstructed, preventing the tongue from being involved in processes that are harmful to health, such as snoring or apnoea.

However, it has been found that none of the existing systems proposes means for correcting tongue position based on the measurement of the vacuum pressure in the oral cavity generated by swallowing, wherein based on said measured vacuum pressure, a study and/or monitoring of the tongue position can be conducted to assist the user in training to correct an inadequate tongue position and at the same time assist a healthcare professional in understanding the position occupied by the tongue and assessing correction alternatives.

### DESCRIPTION OF THE INVENTION

The invention is as defined in the appended claims.

To respond to the gap found in the technical field, the present disclosure proposes a device for determining tongue position based on the measurement of vacuum pressure in a user's oral cavity, said negative pressure being generated by the tongue movement during swallowing, to provide information, for example, in the form of a warning to the user or to healthcare personnel about whether a correct tongue position is achieved based on said negative pressure.

It will be obvious to a person skilled in the art that in the context of the invention a negative pressure in the oral cavity refers to a pressure that is lower than the pressure measured outside said oral cavity which generally corresponds to, but is not limited to, the atmospheric pressure at which a user is using the device.

The negative pressure in the oral cavity is generated by swallowing and, to prevent air from leaking into the oral cavity through the dental interstices, by reducing the vacuum pressure; the device of the invention has an intraoral screen, preferably made of a flexible material suitable for non-invasive, intraoral use for the user, which, as mentioned, assists the user in maintaining negative pressure after the swallowing action.

In addition to the intraoral screen, the device comprises a conduit that can be connected by a first end to the intraoral screen at a connection end arranged therein and by a second end to a pressure gauge, in turn able to be coupled to a base of said device. Such that, in a condition of use of the device by a user, the intraoral screen is arranged between the inner portion of the lips and the front portion of the user's teeth to block the dental interstices and the conduit fluidly communicates the inside of the oral cavity with the pressure gauge, such that the negative pressure generated in the oral cavity by swallowing is measured by the pressure gauge.

Preferably, the conduit will have a saliva trap with a membrane made of an impermeable and preferably flexible material, such as GORE-TEX^{®}, which will prevent saliva from entering inside the conduit itself and subsequently contacting the pressure gauge.

Coupled to the base is an electronic module which comprises a processing unit configured to obtain/calculate/determine data on the tongue position based on the measurement of the vacuum pressure in the oral cavity. The electronic module comprises wireless connection means, for example, a Bluetooth card, or a data connection port that can be coupled to the base and connected to said electronic module prepared to transmit data on the pressure in the oral cavity and/or receive data or information to/from any external terminal, such as a computer, smartphone, tablet or similar.

As described above, a device is proposed that assists in the sealing of the oral cavity in order to measure the negative pressure generated by swallowing, for example when swallowing saliva, wherein by measuring the negative pressure it is possible to identify whether the tongue position is correct, and if it not, it can generate signals, transmit data to the external terminal to assist in training the correct position of the tongue, to create habits that prevent the tongue from obstructing the airways.

For example, a negative pressure of 15m Bar in the oral cavity will indicate that the tongue is in the proper position, occupying the entire oral cavity without any space being able to exist. Therefore, the tongue is fully attached to the palate.

The ideal negative pressure at which the tongue is considered to be in the proper position can be set within a specific range or can be set by medical personnel for a specific user and his or her particular condition.

Thus, with the device it is possible to monitor pressure in the oral cavity and/or changes therein, indicative of the position of the tongue, and said device can also be configured to monitor the time that the negative pressure is maintained in the oral cavity by the user.

In a preferred embodiment, the device comprises means for measuring the vacuum pressure generated by swallowing in an air chamber formed between the dorsum of the tongue and the palate, better known as the space of Donders, wherein such means comprise a flexible tube or cannula that can be connected by a first end to the conduit and/or to the pressure gauge and a second end, opposite the first end, open to the atmosphere, so that in an operating condition of the device, the second end of the tube is arranged in the space of Donders, so that the pressure gauge detects the air pressure in said space of Donders and sends the data on said detection to the processing unit for subsequent processing of such data.

A person skilled in the sector will know that the space of Donders is a virtual space that is generated with respect to the hard palate behind the lip seal and up to the seal between the dorsum of the tongue and the soft palate. A negative pressure is generated in this space, capable of generating a force of up to 0.3 Kg, creating a suction effect. It is an area of air bubbles that, when compressed, facilitate the opening of the lumen for the passage of food towards the pharynx.

Alternatively, a bulb is arranged at the second end of the tube, said bulb being configured to, in an operating condition of the device, be located in the space of Donders and seal said space hermetically or in an airtight manner, to prevent pressure leaks during measurement of the same, making it much more accurate. Moreover, this bulb makes it so that the muscle pressure of the tongue does not modify the measurement of the manometer.

Preferably, the bulb is made from a flexible, soft-touch, unbreakable, and biologically compatible polymeric material.

In another embodiment or indication of use, the device can be used to determine the pressure in the nasopharyngeal space (EPP, EpiPharyngeal Pressure) by measuring the inhalation/exhalation pressure in the nasal cavities of a user's nose, thus becoming a device for determining the nasopharyngeal pressure (EPPI, EpiPharyngeal Pressure Index), wherein the first end of the tube can be connected to the conduit and/or to the pressure gauge, whereas the second end is open to the atmosphere and is intended to be at least partially introduced into a user's nostril where, in an operating condition of this device, the second end is at least partially introduced into one of the nostrils, being coupled in an airtight manner in said nostril, such that the pressure gauge detects the inhalation pressure in the nasopharyngeal space and sends the data on the detection to the processing unit for treatment and analysis.

In an additional embodiment, the foregoing device is fitted to a mask of the type intended to simultaneously cover the user's mouth and nose, such as, for example, FFP2 and FFP3 masks or the like. Even more preferably, the tube can be coupled to the mask by suitable means, for example, passing through the mask through a hole provided therein. This may be useful for measuring breathing during use of these masks which have become popular with the COVID-19 pandemic.

Preferably, a plug is releasably coupled to the second end of the tube, said plug being configured to block in an airtight manner the nasopharyngeal cavity such that, in an operative condition of the device, the plug blocks the nostril so that fluid communication between the pressure gauge and the nostril and/or the nasopharyngeal space is established only through the tube, making the measurement of inhalation pressure more accurate.

The material from which the plug is made must block the nostril without modifying the shape of the tissue inside said nostril so as not to affect the measurement. Therefore, said material should be selected from mouldable, non-porous, soft-touch polymers. According to the foregoing, the connection is completely passive and does not modify the nostril in any way since a soft sponge or similar is used which obstructs without effect on the tissues of the nose.

In this embodiment, the device enables, in addition to measuring and determining pressure in the nasopharyngeal space (EPP) located behind the nasal septum, measuring the pressure/inhalation capacity for each of the nostrils and analysing how the application of a nose decongestant substance affects the ability to inhale/expel air, as well as the effect of a mask covering a user's mouth and nose. In this sense, the device must first be used in one nostril, taking the measurement, and then the device is to be used in the other nostril, taking the measurement. Later, after having applied a dilator, decongestant or some other substance in the nostrils, or having put on the mask, the measurements can be repeated and compared with the initial measurements in order to analyse the effects.

The different embodiments of the described device consist of innovative structural and constituent features heretofore unknown, reasons which, taken together with its usefulness, provide it with sufficient grounds for obtaining the requested exclusivity privilege.

### BRIEF DESCRIPTION OF THE DRAWINGS

To complement the description provided herein, and for the purpose of helping to make the features of the invention more readily understandable, said description is accompanied by a set of drawings constituting an integral part of the same, which by way of illustration and not limitation represents the following:
Figure 1 shows a general view of the device object of the invention
Figure 2 shows an exploded view of the components of the device
Figure 3 shows a detailed view of the components of the electronic card
Figure 4 shows an alternative embodiment of the device for measuring vacuum pressure in the space of Donders or inhalation pressure in the nasopharyngeal space.

### List of references and figures:

- 1.: Intraoral screen
- 2.: Base
- 3.: Conduit
- 4.: Pressure gauge
- 5.: Impermeable membrane
- 6.: Electronic module
- 7.: Wireless connection means
- 8.: Power supply means
- 9.: Data connection port
- 10.: Casing
- 30.: Tube
- 31.: First end of the tube
- 32.: Second end of the tube
- 33.: Bulb
- 34.: Plug

### PREFERRED EMBODIMENT OF THE INVENTION

In the following detailed description of the preferred embodiments, reference is made to the accompanying drawings of this specification and wherein specific preferred embodiments in which the invention may be implemented are shown by way of illustration. These embodiments are described in sufficient detail to enable those skilled in the art to implement the invention, and it is understood that other embodiments may be used and logical structural, mechanical, electrical, and/or chemical changes may be made without departing from the scope of the invention. To avoid details that are not needed by those skilled in the art to implement the detailed description, it should therefore not be taken in a limiting sense.

Specifically, a device is disclosed for determining and modulating the tongue position based on the measurement of vacuum pressure in an oral cavity, which is generated by the tongue movement during the user's swallowing phase, the device comprising:
- an intraoral screen 1 intended to, in use or operation, be arranged in the oral cavity, plugging the dental interstices;
- a base 2 able to be connected to the intraoral screen 1 through at least one conduit 3, configured to, in a condition of use or operation of the device, establish fluid communication between the inside of the oral cavity and a pressure gauge 4 able to be coupled to the base 2, intended to measure the vacuum pressure produced by swallowing;
- and an electronic module 6 able to be coupled to the base 2, said electronic module 6 being in data communication with the pressure gauge 4, and comprising a processing unit configured to obtain data on the tongue position based on the measurement of vacuum pressure in the oral cavity.

In this way, a device capable of determining a user's tongue position is obtained, based on the measurement of the internal pressure of his or her intraoral cavity, thanks to a pressure gauge 4 and the subsequent processing of the data obtained by the same, by means of an electronic module 6.

In a preferred embodiment, the intraoral screen 1 comprises at least one connection end 1A, to which the conduit 3 can be connected, to achieve fluid communication between the inside of the oral cavity and the pressure gauge 4, when the device is in use, measuring the pressure after the user's swallowing process, for example, when swallowing saliva

Preferably, the pressure gauge 4 is a manometer. A person skilled in the art will understand that the term pressure gauge 4 includes all sensitive devices for measuring pressure in the oral cavity and that may be applicable within the context of the invention.

In a preferred embodiment, the intraoral screen 1 will be made of a hydrophobic elastic polymeric material, preferably silicone for intraoral use, so as to achieve adaptability to the morphology of the user's oral cavity and thus seal the oral cavity and prevent air from entering therein when the vacuum has been generated during swallowing.

The preferred position in operation of the intraoral screen 1 will be between the inner portion of the lips and the front portion of the teeth of the user's oral cavity.

Moreover, the electronic module 6 comprises wireless communication means 7, configured to transmit, based on the measurement of the vacuum pressure in the oral cavity, the data on the tongue position towards an external terminal and/or receive information from said external terminal. In such a way that information can be exchanged between the device object of the present invention and said external terminal.

In another preferred embodiment, the device comprises a data connection port 9, able to be coupled to the base 2, which will be able to be connected to the electronic module 6, and wherein the data connection port 9 is configured to transmit data on the tongue position, obtained by measuring the vacuum pressure in the oral cavity, towards the external terminal and/or receive information from said terminal. As a result, information is also exchanged in person, through the direct connection between the data connection port 9 and the external terminal.

As previously mentioned, the electronic module 6 comprises a processing unit configured to obtain data on the tongue position based on the measurement the vacuum pressure in the oral cavity. Such that, given a vacuum pressure established as a reference, for example, 15m Bar (millibar), the processing unit determines whether the tongue position is correct based on the measurement obtained by the pressure gauge 4 of the vacuum pressure generated in the oral cavity and/or also through the time said negative pressure is maintained.

The processing unit can monitor the position of the tongue, as well as the time that the negative pressure is maintained, through the continuous measurement performed by the pressure gauge 4. For example, if the reference vacuum pressure is 15m Bar, and the measured pressure is 10m Bar, this could be insufficient for a proper tongue position, and/or it is also possible that, by having reached the reference pressure, said negative pressure has been sustained for less time than necessary.

According to the last two embodiments, the device comprises both wireless and direct connection communication means for communicating with external devices, successfully exchanging data about the pressure measured in the oral cavity with external terminals, facilitating the processing and treatment of said data.

In a preferred embodiment, the electronic module 6 comprises signalling means configured for emitting a warning signal, wherein the processing unit of the electronic module 6 is configured for, based on the measurement of the negative pressure in the oral cavity, determining an inadequate position of the tongue and transmitting a warning signal through the signalling means.

These signalling means can be in the form of a speaker for sending an acoustic signal, and/or an LED bulb for sending a visual signal, and/or a vibrating mechanism, such as those in smartphones, for sending a vibrating signal, for warning the user of a poor positioning of the tongue inside the oral cavity.

The device comprises power supply means 8, coupleable to the base 2, and configured for supplying power to the electronic module 6 and/or the pressure measurement means 4. Preferably the power supply means 8 will consist of a rechargeable battery detachable from the base 2, such as, for example, a rechargeable battery similar to that of AirPods^{®} by Apple^{®}, or rechargeable batteries of similar devices.

Likewise, in non-illustrated alternative embodiments, the device of the invention comprises additional pressure gauges 4 intended for being coupled to one or more conduits 3 or to other similar conduits (not shown) distributed across the intraoral shield 1. The purpose of arranging these additional pressure gauges 4 is to measure the vacuum pressure in the intraoral compartments, for example, at different points of the intraoral cavity. These additional pressure gauges 4 are coupled to additional conduits 3 suitably distributed across the intraoral shield 1 and are not necessarily arranged at the base 2, although said additional pressure gauges 4 must be in data communication by suitable means with the electronic module 6 for transmitting to the latter the vacuum pressure measurements taken at different points by each of said additional pressure gauges 4.

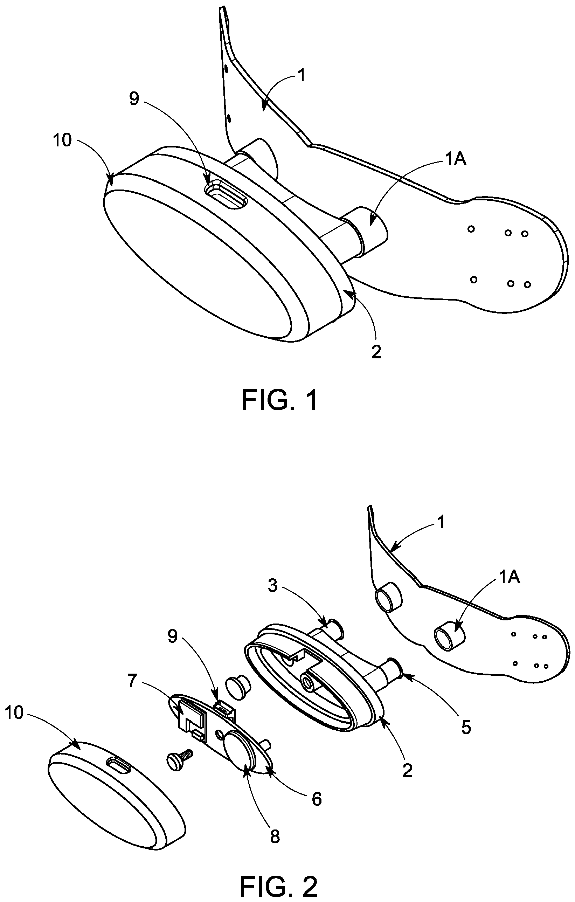

As observed in Figure 1, in a preferred embodiment, the device comprises a casing 10 coupleable to the base 2 and configured for protecting the electronic module 6 and/or the pressure gauge 4 and/or the data communication port 9 and/or the power supply means 8, wherein the casing 10 comprises at least one opening for accessing the data communication port 9, such that the casing 10 protects the mentioned components from external agents.

Moreover, the device comprises at least one detachable membrane 5, preferably made of an impermeable material, such as GoreTex^{®}, arranged inside the conduit 3 and configured for preventing the passage of fluids through said conduit 3 which may come into contact with the pressure gauge 4 and/or the electronic module 6, causing their deterioration or malfunction.

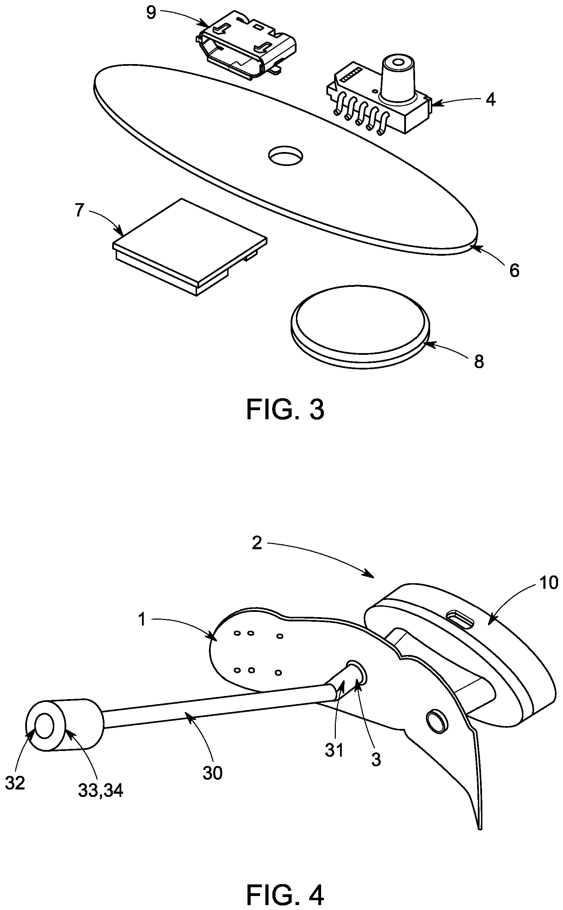

As observed in Figure 4, in an alternative embodiment, the device comprises a tube (30) which can be connectable or be connected in a fixed manner at a first end (31) to the conduit (3) and/or to the pressure gauge (4), also comprising a second end (32), which is opposite the first end (31), said second end being open to the atmosphere, such that in an operating condition of the device, the second end (32) of the tube (30) is arranged in a tight manner in the space of Donders between the tongue and the palate of the user, establishing fluid communication between said space of Donders and the pressure gauge (4), such that the pressure gauge (4) detects a pressure which is generally vacuum pressure in said space and sends the data about this detection to the processing unit for treatment.

As seen in Figure 4, at the second end (32) of the tube (30) there is coupled either in a fixed or in a releasable manner a bulb (33) configured for being located in the space of Donders and sealing said space in a tight manner in an operating condition of the device for preventing pressure leakages that may affect the measurements taken by the device.

The bulb (33) is preferably made from a soft-touch, unbreakable, and biologically compatible flexible polymer material for preventing irritations with parts of the oral cavity with which it comes into contact.

Additionally, by means of the present invention it is possible to take measurement/perform an estimation of the suction pressure in the nasopharyngeal space of the nose of a user.

In this sense, in an operating condition of the device, the second end (32) of the tube (30) is configured for being introduced at least partially into one of the nasal fossae of a user, being arranged in a tight manner in said fossa, such that the pressure gauge (4) detects changes in pressure in the nasopharyngeal space (negative changes during inhalation, positive changes during exhalation) when the air enters and exits through the free nasal fossa, and sends such data about the detection to the processing unit for subsequent treatment.

As seen in Figure 4, at the second end (32) of the tube (30) there is arranged a plug (34) configured for blocking the nasal fossa into which the second end (32) is introduced such that in the operating condition of the device, the plug (34) blocks the nasal fossa such that fluid communication between the pressure gauge (4) and the free nasal fossa, passing through the nasopharyngeal space, is established only through the tube (30).

It is important to take into account that the bio-functional system also includes the nose, which is characterised by its position between the valves (velopharyngeal sphincter) and the nasal entry.

The nasopharynx is adjacent to the valve; this space is divided by the nasal septum into two channels, nasal cavities, the outer openings of which form the two nasal fossae. From the nasal fossa to the nasopharyngeal space directly adjacent to the valve, there are, therefore, two separate functional spaces. The invention takes advantage of this fact to determine the pressure, and/or alternatively, flow of breathable air.

The left and right halves of the nose can be used separately as a channel to measure pressure in the nasopharyngeal space, which is located next to both halves of the nose, behind the septum.

If the valve is closed on one side and half of the nose behind it is understood to be a pressure conducting channel, a line can be connected in a fluid manner to the pressure gauge (4) at the mid-point of the closed nose. This pressure line measures the pressure existing at the end of the nasal side in the nasopharyngeal space.

Therefore, if the oral cavity is closed by the up-lock condition and the valve of the nasal side connected to the measurement tube (30) is also closed, that is, the second end (32) of the tube (30) is connected in a tight manner in the nasal fossa, breathable air must flow exclusively to the pressure gauge through the tube (30).

A mask (not shown) can be used as part of the device and is configured for simultaneously covering the mouth and nose of the user, the tube (30) being coupleable to the mask by suitable means.

The difference in pressure between the environment and the nasopharyngeal space, that is, the rear end of both nasal sides, will be proportional to the resistance of the side nasal, according to Ohm's law for linear flow. In this sense, it is assumed that a test subject generates a constant air flow for breathing at rest. When breathing with normal intensity and frequency, it requires a greater difference in pressure with a greater flow resistance of the nasal membranes through which the flow arrives.

This can be determined by measuring the amplitude of pressure in the nasopharyngeal space, which must increase with flow resistance of the nose, assuming a constant flow while breathing at rest.

Taking this measurement sequentially on each side of the nose yields result that is useful for diagnostic purposes, being obtained in little time by means of the sequential measurement of the difference in pressure in both nasal fossae, determining pressure in the nasopharyngeal space.

Measurement contour conditions are kept constant by means of the up-lock manoeuvre), such that the secondary air is excluded through the oral cavity towards the nasopharynx. By incorporating the use of the up-lock activator during measurements, pressure in the nasopharynx being caused by a secondary flow through the mouth to the throat is ruled out.

Likewise, the present disclosure
proposes a connectable external terminal in communication with the device of the preceding embodiments, which is configured for receiving data about the position of the tongue obtained by the device, and/or data about the suction pressure in the nasopharyngeal space, and/or determining which of the nasal fossae offers the greatest air suction resistance as described heretofore, wherein the external terminal comprises processing means configured for determining whether or not the position of the tongue is correct based on the obtained data about the position of the tongue, and/or whether the suction pressure in the nasopharyngeal space, and/or the aspiration resistance by each of the nasal fossae, and generating a warning signal through said external terminal for the user of the device of the preceding embodiments.

## Claims

1. A device for assessing a correct position of a user's tongue, comprising an intraoral shield (1) intended for being arranged in the oral cavity of the user and configured to block interdental spaces of the teeth in said oral cavity, a conduit (3) configured to be connected at a first end to the intraoral shield (1) and at a second end to a pressure gauge (4), said pressure gauge (4) in turn being couplable to a base (2), wherein the conduit (3) is configured to establish fluid communication between the interior of the oral cavity and the pressure gauge (4) and said pressure gauge (4) is configured to measure the negative pressure generated in the oral cavity due to deglutition, and an electronic module (6) couplable to the base (2), said electronic module (6) being in data communication with the pressure gauge (4) and comprising a processing unit configured to assess the correct position of the tongue by comparing the data of the pressure measured by the pressure gauge (4) and a pre-established pressure; the device **characterised in that** it comprises a tube (30) configured to be connected at a first end (31) to the conduit (3) and comprising a second end (32), opposite the first end (31), wherein the second end (32) is configured to be arranged in tight manner in the Donders space located between the tongue and the palate of the user, wherein at the second end (32) of the tube (30) is provided a bulb (33) configured to be located in the space of Donders and sealing said space in a tight manner, such that the pressure gauge (4) detects a vacuum pressure in said Donders space and sends the data about this detection to the processing unit for the assessment of the correct position of the tongue.

2. The device according to any of the preceding claims, wherein the intraoral shield (1) is made from a hydrophobic elastic polymer material.

3. The device according to any of the preceding claims, wherein the intraoral shield (1) is configured to be interposed between an inner part of the lips and a frontal part of the teeth in the oral cavity.

4. The device according to any of the preceding claims, wherein the electronic module (6) comprises wireless communication means (7) configured for transmitting data about the vacuum pressure in the oral cavity to an external terminal and/or receiving information from said external terminal.

5. The device according to any of the preceding claims comprising a data communication port (9) couplable to the base (2) and connectable to the electronic module (6), wherein the data communication port (9) is configured for transmitting data about the vacuum pressure in the oral cavity to the external terminal and/or receiving information from said external terminal.

6. The device according to any of the preceding claims, wherein the electronic module (6) comprises signalling means configured for emitting a warning signal, wherein the processing unit of the electronic module (6) is configured for, based on the position of the tongue determined from the measurement of the vacuum pressure, determining an inadequate position of the tongue and transmitting a warning signal through the signalling means.

7. The device according to any of the preceding claims, comprising power supply means (8) arranged in the base (2), configured for supplying power to the electronic module (6) and/or the pressure gauge (4).

8. The device according to preceding claim, wherein the power supply means (8) comprise a rechargeable battery detachable from the base (2).

9. The device according to claim 7, comprising a casing (10) couplable to the base (2), configured to protect the electronic module (6) and/or the pressure gauge (4) and/or the data communication port (9) and/or the power supply means (8), wherein the casing (10) comprises an opening for accessing the data communication port (9).

10. The device according to any of the preceding claims, comprising at least one membrane (5) arranged inside the conduit (3), configured to prevent the passage of fluids through the conduit (3).

11. The device according to the preceding claim, wherein the membrane (5) is impermeable and interchangeable.

12. The device according to any of the preceding claims, comprising one or more pressure gauges (4) coupled to one or more conduits (3).

13. The device according to any of the preceding claims wherein the second end (32) of the tube (30) is further configured to be introduced at least partially into one of the nasal fossae of the nose of a user arranged in a tight manner in the nasal fossae, such that the pressure gauge (4) detects the suction pressure in the nasopharyngeal space and sends data about the detection to the processing unit.

14. The device according to preceding claim, comprising a mask configured for simultaneously covering the mouth and the nose of the user, the tube (30) being couplable to the mask.

15. The device according to claims 13 or 14 further comprises a plug (34) arranged at the second end (32) of the tube (30) configured to block the nasal fossa in such a way that the air in said fossa flows through only the tube (30).

## Patentansprüche

1. Vorrichtung zum Beurteilen einer korrekten Position der Zunge eines Benutzers, umfassend eine intraorale Blende (1), die dazu vorgesehen ist, in der Mundhöhle des Benutzers angeordnet zu werden, und die so konfiguriert ist, dass sie die Zahnzwischenräume der Zähne in der Mundhöhle blockiert, eine Leitung (3), die so konfiguriert ist, dass sie an einem ersten Ende mit der intraoralen Blende (1) und an einem zweiten Ende mit einem Druckmesser (4) verbunden ist, wobei der Druckmesser (4) wiederum an eine Basis (2) koppelbar ist, wobei die Leitung (3) so konfiguriert ist, dass sie eine Fluidkommunikation zwischen dem Inneren der Mundhöhle und dem Druckmesser (4) herstellt, und der Druckmesser (4) so konfiguriert ist, dass er den in der Mundhöhle beim Schlucken erzeugten Unterdruck misst, und ein elektronisches Modul (6), das an die Basis (2) koppelbar ist, wobei das elektronische Modul (6) in Datenkommunikation mit dem Druckmesser (4) steht und eine Verarbeitungseinheit umfasst, die so konfiguriert ist, dass sie die korrekte Position der Zunge durch Vergleichen der Daten des von dem Druckmesser (4) gemessenen Drucks mit einem zuvor ermittelten Druck bewertet, die Vorrichtung **dadurch gekennzeichnet ist, dass** sie einen Schlauch (30) F umfasst, der so konfiguriert ist, dass er an einem ersten Ende (31) mit der Leitung (3) verbunden ist und ein zweites Ende (32) gegenüber dem ersten Ende (31) umfasst, wobei das zweite Ende (32) so konfiguriert ist, dass es im Donders'schen Raum, der sich zwischen der Zunge und dem Gaumen des Benutzers befindet, eng anliegend angeordnet ist, wobei am zweiten Ende (32) des Schlauchs (30) ein Kolben (33) bereitgestellt ist, der so konfiguriert ist, dass er sich im Donders'schen Raum befindet und den Raum dicht verschließt, sodass der Druckmesser (4) einen Unterdruck im Donders'schen Raum erfasst und die Daten über diese Erfassung an die Verarbeitungseinheit zur Beurteilung der korrekten Position der Zunge sendet.

2. Vorrichtung nach einem der vorhergehenden Ansprüche, wobei die intraorale Blende (1) aus einem hydrophoben, elastischen Polymermaterial hergestellt ist.

3. Vorrichtung nach einem der vorhergehenden Ansprüche, wobei die intraorale Blende (1) so konfiguriert ist, dass sie zwischen einem inneren Teil der Lippen und einem vorderen Teil der Zähne in der Mundhöhle eingefügt ist.

4. Vorrichtung nach einem der vorhergehenden Ansprüche, wobei das elektronische Modul (6) drahtlose Kommunikationsmittel (7) umfasst, die so konfiguriert sind, dass sie Daten über den Unterdruck in der Mundhöhle an ein externes Endgerät übertragen und/oder Informationen von dem externen Endgerät empfangen.

5. Vorrichtung nach einem der vorhergehenden Ansprüche, die einen Datenkommunikationsanschluss (9) umfasst, der an die Basis (2) gekoppelt und mit dem elektronischen Modul (6) verbunden werden kann, wobei der Datenkommunikationsanschluss (9) so konfiguriert ist, dass er Daten über den Vakuumdruck in der Mundhöhle an das externe Endgerät überträgt und/oder Informationen von dem externen Endgerät empfängt.

6. Vorrichtung nach einem der vorhergehenden Ansprüche, wobei das elektronische Modul (6) Signalgebungsmittel umfasst, die so konfiguriert sind, dass sie ein Warnsignal aussenden, wobei die Verarbeitungseinheit des elektronischen Moduls (6) so konfiguriert ist, dass sie auf der Grundlage der aus der Messung des Vakuumdrucks bestimmten Position der Zunge eine inadäquate Position der Zunge bestimmt und ein Warnsignal über die Signalgebungsmittel übermittelt.

7. Vorrichtung nach einem der vorhergehenden Ansprüche, die in der Basis (2) angeordnete Stromversorgungsmittel (8) umfasst, die so konfiguriert sind, dass sie das elektronische Modul (6) und/oder den Druckmesser (4) mit Strom versorgen.

8. Vorrichtung nach dem vorhergehenden Anspruch, wobei die Stromversorgungsmittel (8) eine wiederaufladbare Batterie umfassen, die von der Basis (2) abnehmbar ist.

9. Vorrichtung nach Anspruch 7, die ein an die Basis (2) koppelbares Gehäuse (10) umfasst, das so konfiguriert ist, dass es das elektronische Modul (6) und/oder den Druckmesser (4) und/oder den Datenkommunikationsanschluss (9) und/oder die Stromversorgungsmittel (8) schützt, wobei das Gehäuse (10) eine Öffnung für den Zugang zum Datenkommunikationsanschluss (9) umfasst.

10. Vorrichtung nach einem der vorhergehenden Ansprüche, die mindestens eine Membran (5) umfasst, die im Inneren der Leitung (3) angeordnet ist und so konfiguriert ist, dass sie den Durchtritt von Flüssigkeiten durch die Leitung (3) verhindert.

11. Vorrichtung nach dem vorhergehenden Anspruch, wobei die Membran (5) undurchlässig und austauschbar ist.

12. Vorrichtung nach einem der vorhergehenden Ansprüche, die einen oder mehrere Druckmesser (4), die an eine oder mehrere Leitungen (3) gekoppelt sind, umfasst.

13. Vorrichtung nach einem der vorhergehenden Ansprüche, wobei das zweite Ende (32) des Schlauchs (30) ferner so konfiguriert ist, dass es mindestens teilweise in eine der Nasenhöhlen der Nase eines Benutzers eingeführt und in den Nasenhöhlen eng anliegend angeordnet wird, sodass der Druckmesser (4) den Saugdruck im Nasenrachenraum erfasst und Daten über die Erfassung an die Verarbeitungseinheit sendet.

14. Vorrichtung nach dem vorhergehenden Anspruch, die eine Maske umfasst, die so konfiguriert ist, dass sie gleichzeitig den Mund und die Nase des Benutzers bedeckt, wobei der Schlauch (30) an die Maske koppelbar ist.

15. Vorrichtung nach Anspruch 13 oder 14, die ferner einen Stopfen (34) umfasst, der am zweiten Ende (32) des Schlauchs (30) angeordnet ist und so konfiguriert ist, dass er die Nasenhöhle in einer Weise blockiert, dass die Luft in der Höhle nur durch den Schlauch (30) strömt.

## Revendications

1. Dispositif permettant d'évaluer la position correcte de la langue d'un utilisateur, comprenant un écran intrabuccal (1) destiné à être agencé dans la cavité buccale de l'utilisateur et conçu pour bloquer les espaces interdentaires des dents dans ladite cavité buccale, un conduit (3) conçu pour être raccordé au niveau d'une première extrémité au bouclier intrabuccal (1) et au niveau d'une seconde extrémité à un manomètre (4), ledit manomètre (4) pouvant à son tour être accouplé à une base (2), dans lequel le conduit (3) est conçu pour établir une communication fluidique entre l'intérieur de la cavité buccale et le manomètre (4) et ledit manomètre (4) est conçu pour mesurer la pression négative générée dans la cavité buccale en raison de la déglutition, et un module électronique (6) pouvant être accouplé à la base (2), ledit module électronique (6) étant en communication de données avec le manomètre (4) et comprenant une unité de traitement conçue pour évaluer la position correcte de la langue en comparant les données de la pression mesurée par le manomètre (4) et une pression préétablie ; le dispositif **caractérisé en ce qu'**il comprend un tube (30) conçu pour être raccordé au niveau d'une première extrémité (31) au conduit (3) et comprenant une seconde extrémité (32), opposée à la première extrémité (31), dans lequel la seconde extrémité (32) est conçue pour être agencée de manière étanche dans l'espace de Donders situé entre la langue et le palais de l'utilisateur, dans lequel au niveau de la seconde extrémité (32) du tube (30) est prévue une ampoule (33) conçue pour être située dans l'espace de Donders et sceller ledit espace de manière étanche, de telle sorte que le manomètre (4) détecte une pression de vide dans ledit espace de Donders et envoie les données concernant cette détection à l'unité de traitement pour l'évaluation de la position correcte de la langue.

2. Dispositif selon l'une quelconque des revendications précédentes, dans lequel le bouclier intrabuccal (1) est constitué d'un matériau polymère élastique hydrophobe.

3. Dispositif selon l'une quelconque des revendications précédentes, dans lequel le bouclier intrabuccal (1) est conçu pour être interposé entre une partie interne des lèvres et une partie frontale des dents dans la cavité buccale.

4. Dispositif selon l'une quelconque des revendications précédentes, dans lequel le module électronique (6) comprend des moyens de communication sans fil (7) conçus pour transmettre des données concernant la pression de vide dans la cavité buccale à un terminal externe et/ou recevoir des informations dudit terminal externe.

5. Dispositif selon l'une quelconque des revendications précédentes comprenant un port de communication de données (9) pouvant être accouplé à la base (2) et pouvant être raccordé au module électronique (6), dans lequel le port de communication de données (9) est conçu pour transmettre des données concernant la pression de vide dans la cavité buccale au terminal externe et/ou recevoir des informations dudit terminal externe.

6. Dispositif selon l'une quelconque des revendications précédentes, dans lequel le module électronique (6) comprend des moyens de signalisation conçus pour émettre un signal d'avertissement, dans lequel l'unité de traitement du module électronique (6) est conçue pour, en fonction de la position de la langue déterminée à partir de la mesure de la pression de vide, déterminer une position inadéquate de la langue et transmettre un signal d'avertissement par l'intermédiaire des moyens de signalisation.

7. Dispositif selon l'une quelconque des revendications précédentes, comprenant des moyens d'alimentation en puissance (8) agencés dans la base (2), conçus pour alimenter en puissance le module électronique (6) et/ou le manomètre (4).

8. Dispositif selon la revendication précédente, dans lequel les moyens d'alimentation en puissance (8) comprennent une batterie rechargeable détachable de la base (2).

9. Dispositif selon la revendication 7, comprenant un boîtier (10) pouvant être accouplé à la base (2), conçu pour protéger le module électronique (6) et/ou le manomètre (4) et/ou le port de communication de données (9) et/ou le moyen d'alimentation en puissance (8), dans lequel le boîtier (10) comprend une ouverture permettant d'accéder au port de communication de données (9).

10. Dispositif selon l'une quelconque des revendications précédentes, comprenant au moins une membrane (5) agencée à l'intérieur du conduit (3), conçue pour empêcher le passage de fluides à travers le conduit (3).

11. Dispositif selon la revendication précédente, dans lequel la membrane (5) est imperméable et interchangeable.

12. Dispositif selon l'une quelconque des revendications précédentes, comprenant un ou plusieurs manomètres (4) accouplés à un ou plusieurs conduits (3).

13. Dispositif selon l'une quelconque des revendications précédentes dans lequel la seconde extrémité (32) du tube (30) est en outre conçue pour être introduite au moins partiellement dans l'une des fosses nasales du nez d'un utilisateur agencé d'une manière étanche dans les fosses nasales, de telle sorte que le manomètre (4) détecte la pression d'aspiration dans l'espace nasopharyngé et envoie des données concernant la détection à l'unité de traitement.

14. Dispositif selon la revendication précédente, comprenant un masque conçu pour couvrir simultanément la bouche et le nez de l'utilisateur, le tube (30) pouvant être accouplé au masque.

15. Dispositif selon les revendications 13 ou 14 comprend en outre un bouchon (34) agencé au niveau de la seconde extrémité (32) du tube (30) conçu pour obstruer la fosse nasale de telle manière que l'air dans ladite fosse ne s'écoule qu'à travers le tube (30).
